# EUROPÄISCHE PATENTANMELDUNG

(11) **EP 2 078 515 A1**
(43) Veröffentlichungstag der Anmeldung: **15.07.2009**
(21) Anmeldenummer: 09150123.9
(22) Anmeldetag: 06.01.2009
(51) Int. Cl.: A61F 5/448

(54) **Vorrichtung und Verfahren zur Unterstützung des Verbindens eines Auffangbeutels mit einem Stomaverschluß**

(30) Priorität: 11.01.2008 DE 102008004175
(71) Anmelder: Redlich, Hartmut, 69427 Mudau (DE)
(72) Erfinder: Redlich, Hartmut, 69427 Mudau (DE)
(74) Vertreter: Kewitz, Ansgar

(57) **Zusammenfassung**

Vorrichtung zur Unterstützung des Verbindens eines Auffangbeutels (6) mit einem Stomaverschluss, wobei der Stomaverschluss einen Schließring (5) aufweist, der auf einer Basis (4) befestigt ist, gekennzeichnet durch flächige Scheiben (2), die sich unter den Schließring legen oder mit dem Schließring eine stabile, lösbare Verbindung eingehen, wobei die flächigen Scheiben so ausgebildet sind, dass ein kräftiger Druck beim Anbringen des Auffangbeutels ermöglicht wird, da die Scheiben stabil und flächig genug sind, den Druck flächig auf die Bauchdecke zu verteilen, so dass die Bauchdecke nicht einseitig eingedrückt wird.

## Beschreibung

Die Erfindung betrifft eine Vorrichtung und Verfahren zur Unterstützung des Verbindens eines Auffangbeutels mit einem Stomaverschluß. Insbesondere betrifft die Erfindung ein Unterstützungselement (Stoma-Schließhilfe), das ein sicheres Andrücken des Auffangbeutels auf einen Schließring des Stomaverschlusses erlaubt.

### Beschreibung:

Die Erfindung betrifft eine medizinische Versorgung nach Anlage eines Darmausganges (Kolostomie/ Jleostomie). Es versteht sich, dass die Erfindung nicht auf den Darmausgang beschränkt ist, sondern ebenfalls für die Urostomi-Versorgung angewendet werden kann. Bei dieser handelt es sich um einen künstlichen Blasenausgang der in gleicher Weise wie beim Stoma mit Basisplatte und einem Beutel ausgestattet ist. Im Folgenden ist der Begriff Stoma sowohl für beide Anwendungsfälle zu verstehen und nicht beschränkt zu betrachten.

Die medizinische Versorgung besteht im Wesentlichen aus einer Basisplatte und einem Auffangbeutel für den Stuhl, welche mit einem Schließringsystem verbunden werden. Dieses Schließringsystem besteht aus einem flexiblen Klebpflaster, das mit einem stabileren Schließring versehen ist, auf den der Auffangbeutel geklemmt wird.

Bei den üblichen Stomaverschlüssen muss man entweder den Schließring der Basisplatte mit den Fingern anheben, was bei einer frisch angelegten Platte zur Abhebung der Klebefläche um das Stoma führen kann, oder es muss ein starker Druck auf den Schließring ausgeübt werden, um den Verschluss anzubringen, wobei die Bauchdecke eingedrückt wird, was zumindest nach einer Operation nicht angenehm ist.

Die DE 41 18 315 zeigt ein Verschlusssystem einer Basisplatte, an der ein Stomabeutel befestigt wird, wobei im Verbindungsbereich am Stomabeutel ein umlaufender Flansch aus elastischem Material angeordnet ist, und der Stomabeutel durch eine Umlaufende Verzahnung mit der Basisplatte verbunden wird. Der umlaufende Flansch vereinfacht durch Bewegung/Verformung ein Auslösen der Basisplatte aus der Verzahnung.

Die DE 1955531 U zeigt ein Verschlusssystem mit einer Basisplatte für einen Stomabeutel, wobei die Basisplatte durch einen Gürtel an der Bauchdecke gehalten wird. Im Gegensatz zu den Klebetechniken, die am häufigsten anzutreffen sind, muss ein elastischer Gürtel um den Körper geführt werden, an dem die Basisplatte befestigt ist.

Auch die DE 841 197 zeigt ein Verschlusssystem mit einer Basisplatte für einen Stomabeutel, wobei die Basisplatte durch einen Gürtel an der Bauchdecke gehalten wird. Als Stomabeutel kommt eine Pelotte aus Glas zum Einsatz, die durch ein Schniergelenk an der Basisplatte befestigt wird.

Die US 2007 0135783 A1 zeigt ein Schutzschild für ein Stoma mit einer Aussparung, die durch einen Gürtel gehalten wird.

### Überblick über die Erfindung:

Aufgabe der vorliegenden Erfindung ist eine einfachere Befestigung des Auffangbeutels des Stomaverschlusses.

Gelöst wird die Aufgabe durch eine Vorrichtung und ein Verfahren mit den Merkmalen der unabhängigen Ansprüche.

Im Wesentlichen handelt es sich um eine einen Gegendruck ausübende flächige Schließhilfe, die einen Schließring einer Basisplatte untergreift, und dadurch einen kräftigeren Druck beim Anbringen des Beutels gestattet, da die Schließhilfe stabil genug ist, den Druck flächig auf die Bauchdecke zu verteilen, so dass die Bauchdecke nicht einseitig eingedrückt wird.

In der bevorzugten Ausführungsform besteht die Stoma-Schließhilfe aus mindestens zwei großflächigen Platten, die gegeneinander verschiebbar sind, so dass sie den Schließring der Basisplatte untergreifen und in dieser Position bleiben, bis der Auffangbeutel auf den Stomaverschluss gedrückt wurde. Nach dem Anbringen des Beutels wird die Schließhilfe an den beiden Scheiben auseinander gezogen und abgenommen.

Auch zum Stomabeutel wechseln wird die Schließhilfe unter den Schließring der Basisplatte geschoben. Der Stomabeutel wird abgehoben und das Stoma im Bereich des Schließringes gereinigt. Dann wird der neue Stomabeutel wieder aufgedrückt und die Stoma-Schließhilfe abgenommen.

In einer alternativen Ausführungsform gehen die Platten mit dem Schließring eine stabile lösbare Verbindung ein. Dies kann z.B. durch eine Nut- und Federverbindung erfolgen. Alternative Einrastansätze oder Einhakansätze sind denkbar. Jedoch ist sicherzustellen, dass diese ausreichend den Anpressdruck aufnehmen und diesen auf die Scheiben/Platten verteilen, die wiederum den Druck auf die Bauchdecke abgeben.

Die Nut und Zapfen/Federverbindung kann in unterschiedlicher Form erfolgen. Die Nut kann dabei im Schließring und der Zapfen/Feder in den flächigen Scheiben ausgebildet sein, oder die Nut ist in den flächigen Scheiben und der Feder/Zapfen im Schließring ausgebildet. Es kann auch ein bereichsweises Umgreifen des Schließringes durch eine umlaufende Nut erfolgen, ohne dass der Schließring einen expliziten Zapfen aufweist.

In der bevorzugten Ausführungsform umfasst die Vorrichtung mindestens zwei flächige Scheiben, die gegeneinander durch ein Gelenk oder Führung verschoben werden können, so dass sie mit dem Schließring der Basisplatte in eine stabile Druckverbindung treten. In dieser Position verbleiben die Scheiben, bis der Auffangbeutel auf den Stomaverschluss gedrückt wurde. Nach dem Aufdrücken können die Scheiben wieder auseinander gezogen bzw. geschwenkt werden.

In einer weiteren Ausführungsform umfasst die Erfindung mindestens zwei sich überlappende, beliebig geformte Scheiben die miteinander so verbunden werden, dass sie einen runden Ausschnitt bilden, der unter bzw. um den an der Basisplatte befestigten Schließring greifen kann. Hierbei weisen die Scheiben jeweils an einem Randbereich eine halbkreisförmige Aussparung auf und sind so durch ein Gelenk miteinander verbunden, dass die Scheiben gegeneinander verschwenkt werden können, so dass die beiden halbkreisförmigen Aussparungen in einer Stellung eine kreisförmig Öffnung bilden.

In der bevorzugten Ausführungsform sind die Scheiben jeweils sichelförmig ausgebildet. Die Scheiben sind jeweils an einem Ende der Sichel durch ein Gelenk (z. B. eine Niete) miteinander verbunden, so dass sie ein Zusammenführen der Sicheln zu einem Ring ermöglichen. Hierbei liegen die Scheiben bereichsweise übereinander und die Niete erstreckt sich durch beide Scheiben.

Für eine bessere Hygiene bestehen die Scheiben aus abwaschbarem Material wie Kunststoff oder Metall.

Die Einzelnen Anwendungsschritte für den Gebrauch dieser Stoma- Schließhilfe, umfassen das Aufschwenken oder Auseinanderziehen der Stoma- Schließhilfe, die aus gegeneinander beweglichen, flächigen Scheiben bestehen, so dass die Stoma- Schließhilfe um oder unter den Schließring geführt werden kann. Das Zusammenschwenken oder Zusammenschieben der Schließhilfe erfolgt so, dass sie sich unter den Schließring legen, oder mit dem Schließring eine stabile lösbare Verbindung eingehen, wobei die flächigen Scheiben so ausgebildet sind, dass ein kräftigerer Druck beim Anbringen des Auffangbeutels ermöglicht wird, da die Scheiben stabil und flächig genug sind, den Druck flächig auf die Bauchdecke zu verteilen, so dass die Bauchdecke nicht einseitig eingedrückt wird; Nach dem Anbringen des Auffangbeutels erfolgt das Aufschwenken oder Auseinanderziehen der Stoma-Schließhilfe, zum Wegführen der Stoma- Schließhilfe vom Schließring.

Im Folgenden wird ein kurzer Überblick über die Figuren gegeben.

Es zeigt:
Fig. 1a, 1b zeigen die Stoma- Schließhilfe in der Seitenansicht, die sich unter den Schließring erstreckt, sowie eine Draufsicht.
Fig. 2 zeigt eine alternative Ausführungsform der Stoma-Schließhilfe, die Handgriffe rechts und links in geschlossener Form aufweist.
Fig.3 zeigt die Ausführungsform in aufgeschwenktem Zustand.

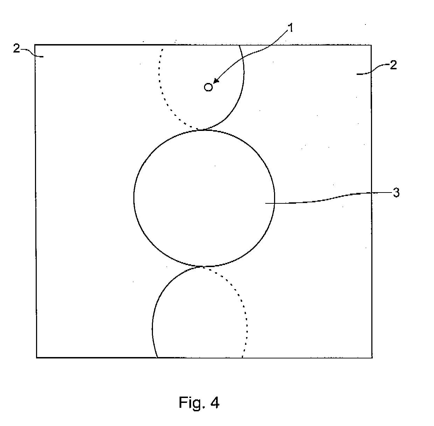

Fig. 4 zeigt eine Ausführungsform in eckiger Form.
Fig. 5a-5c zeigen die unterschiedlichen Möglichkeiten der Verbindung der Schließhilfe mit dem Schließring.
Fig. 6 zeigt einen einstückig ausgebildeten Schließhilfe, der U-förmig ausgebildet ist.

### Detaillierte Beschreibung der Ausführungsformen:

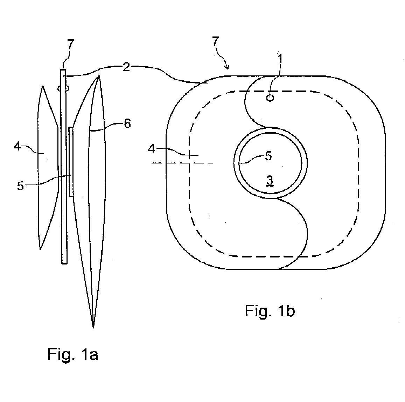

Die Figur 1 zeigt eine Stoma- Schließhilfe 7 in der Seitenansicht, die zwischen einer Basisplatte 4 und einem Schließring 5 angeordnet ist. Die Basisplatte 4 ist über den Stoma- Ausgang geklebt und in der Regel somit auf der Bauchdecke befestigt. Ein Auffangbeutel 6 wird auf den Schließring 5 geklemmt, so dass der Darm sich in dem Auffangbeutel entleeren kann.

Wie der Figur 1b zu entnehmen ist, besteht die Stoma-Schließhilfe 7 aus zwei Scheiben 2, die durch ein Gelenk 1 miteinander drehbar verbunden sind. In der Mitte der Scheiben ist eine Aussparung bzw. Ausschnitt 3, die sich um den Schließring 5 erstreckt. Die beiden Scheiben 2 sind sichelförmig ausgebildet, so dass sie im geschlossenen Zustand einen Ring bilden. Damit der Ring korrekt zusammengeführt werden kann ist ein Gelenk 1 vorgesehen, um das die sichelförmigen Scheiben gedreht werden können. Es ist auch denkbar, dass die Scheiben über ein Führungsschienen- System miteinander verbunden sind, dass es ermöglicht, die Scheiben parallel auseinander zu ziehen, um dann die Stoma-Schließhilfe um bzw. unter den Schließring 5 der Basisplatte 4 zu führen, wobei danach die Scheiben wieder zusammengeschoben werden. Diese alternative Ausführungsform ist nicht als Figur dargestellt.

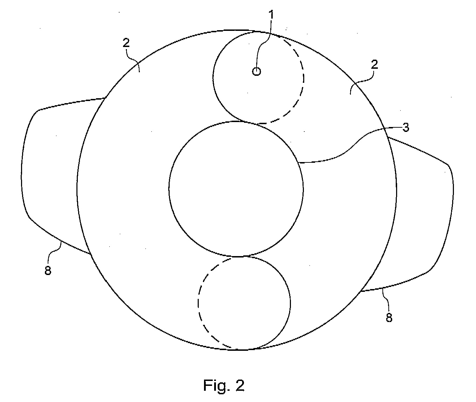

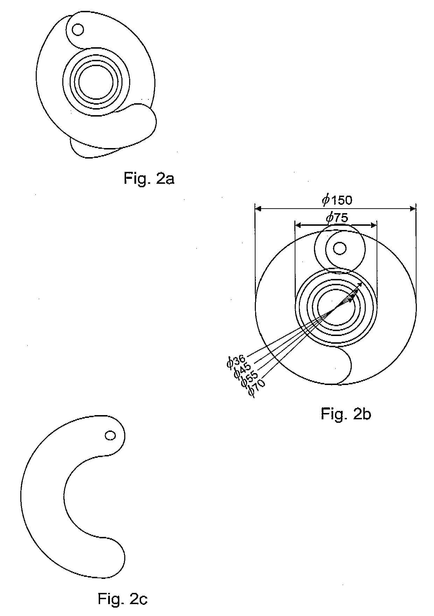

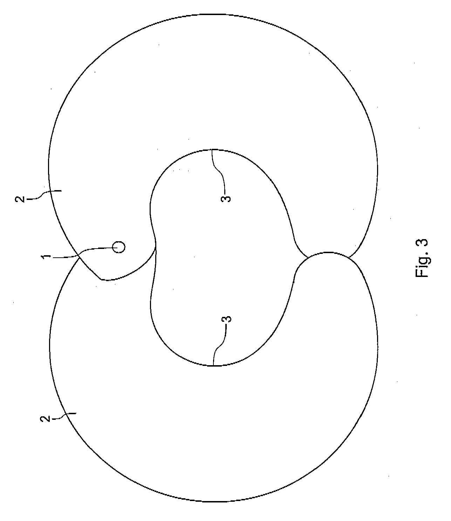

In der Figur 2 sieht man eine alternative Ausführungsform, wobei die Platten jeweils mit Griffen 8 versehen sind, so dass ein Auseinanderziehen und Zusammenschieben vereinfacht wird. In Figur 3 sieht man die Stoma- Schließhilfe in einem geöffneten Zustand, so dass die Aussparung bzw. der Ausschnitt 3 geöffnet wurde, wodurch es ermöglicht wird die Schließhilfe um den Schließring 5 bzw. unter diesen zu führen. Aufgrund des Umstandes, dass die Schließhilfe aus stabilem Material gefertigt ist, wird der Druck beim Aufdrücken des Auffangbeutels gleichmäßig über die ganze Bauchdecke verteilt, so dass ein Verrutschen bzw. ein Abreißen der Basisplatte vermieden werden kann.

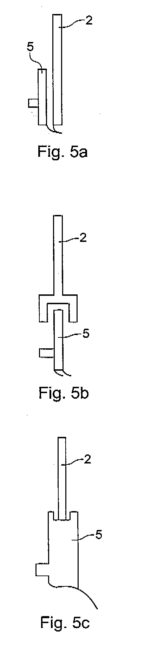

Die Figuren 5a bis 5c zeigen unterschiedliche Formen der Verbindung der Scheiben 2 mit dem Schließring 5. So kann die Scheibe 2, wie in der Figur 5A, dargestellt ist, unter den Schließring 5 geführt werden. Alternativ kann die Scheibe 2 eine umlaufende Nut in ihrem Ausschnitt 3 aufweisen, der sich dann um die Basisplatte 4 erstreckt und somit eine feste Verbindung mit dieser eingeht. In einer weiteren alternativen Ausführungsform, wie sie in Figur 5c dargestellt ist, weist der Schließring 5 eine Nut auf in die die Scheibe 2 geführt wird. Somit entsteht eine Federnutverbindung, die einen festen Halt zwischen beiden Komponenten erlaubt, der auf Wunsch lösbar ist, nachdem der Auffangbeutel aufgedrückt wurde oder entfernt wurde.

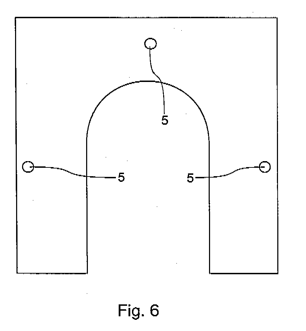

Die Figur 6 zeigt einen einstückig ausgebildete Schließhilfe, die sich um den Schließring erstreckt. Diese ist ohne Gelenk ausgebildet. Ferner weist sie eine U-förmige Aussparung auf, die um bzw. unter den Schließring geschoben wird. Vorzugsweise ist die Platte flexiblen aufgebaut. Dies kann durch Kunststoff oder Papier erfolgen, das entsprechende Eigenschaften aufweist. In einer möglichen Ausführungsform ist die Schließhilfe Teil der Verpackung des Auffangbeutels. So kann der Auffangbeutel aus der Umverpackung entnommen werden und diese kann entlang von perforierten Löchern zerlegt werden, so dass die abgebildete U-förmige Schließhilfe entsteht. In einer möglichen Ausführungsform ist diese mit Magneten versehen, so dass diese auf einem handelsüblichen Stomagürtel befestig werden kann.

### Bezugszeichenliste

1. Gelenk
2. Scheiben
3. Ausschnitt
4. Basisplatte
5. Schließring
6. Auffangbeutel
7. Stoma- Schließhilfe
8. Handgriffe

## Patentansprüche

1. Vorrichtung zur Unterstützung des Verbindens eines Auffangbeutels mit einem Stomaverschluß, wobei der Stomaverschluss einen Schließring aufweist, der auf einer Basisplatte befestigt ist, wobei die Basisplatte auf der Bauchdecke um ein Stoma eines Benutzers befestigbar ist, **gekennzeichnet, durch** mindestens zwei gegeneinander **durch** ein Gelenk verschwenkare oder **durch** eine Führung zueinander verschiebbare Scheiben, die im zueinander bewegten Zustand das Stoma eines Benutzers umgreifend entweder zwischen Schließring und Stomabasisplatte anordenbar oder mit dem Schließring lösbar verbindbar sind, wobei die Scheiben über die äußeren Ränder der Basisplatte hinaus ragen und so stabil ausgebildet ist, dass angreifbare Kräfte beim Wechsel des Auffangbeutels auf die Gesamtfläche der Schreibe verteilt werden."

2. Vorrichtung nach Anspruch 1, wobei die lösbare Verbindung durch eine Nut- und Zapfenverbindung erfolgt, die Nut sich am Schließring und der Zapfen an den Scheiben oder die Nut sich an den Scheiben und der Zapfen am Schließring befindet."

3. Vorrichtung nach einem oder mehreren der vorhergehenden Ansprüche **dadurch gekennzeichnet, dass** sich die Scheiben teilweise überlappen.

4. Vorrichtung nach einem oder mehreren der vorhergehenden Ansprüche **dadurch gekennzeichnet, dass dadurch gekennzeichnet, dass** die Scheiben gegeneinander verschwenkbar sind und jeweils eine halbkreisförmige oder sichelförmige Aussparung aufweisen, wobei sich die halbkreisförmigen oder sichelförmigen Aussparungen im Gebrauchszustand zu einer, das Stoma eines Benutzers umfassenden, kreisförmigen Durchgangsöffnung ergänzen.

5. Vorrichtung nach einem oder mehreren der vorhergehenden Ansprüche **dadurch gekennzeichnet, dass** die Scheiben abwaschbar sind und aus Kunststoff oder Metall bestehen.

6. Vorrichtung nach einem oder mehreren der vorhergehenden Ansprüche **dadurch gekennzeichnet, dass** die Scheiben aus Pappe oder Papier besteht, und vorzugsweise das Papier ein Teil der Verpackung des Auffangbeutels ist.

7. Verfahren zur Unterstützung des Verbindens eines Auffangbeutels mit einer Basisplatte durch einen Stomaverschluss, der einen an der Stomabasisplatte befestigten Schließring aufweist durch eine Schließhilfe, die aus mindestens zwei gegeneinander durch ein Gelenk verschwenkbare oder durch eine Führung zueinander verschiebbare Schreiben gebildet ist, mit folgenden Schritten:
a. Öffnen der Schließhilfe durch Auseinander bewegen der Scheiben,
b. Anlegen der Schließhilfe um das Stoma durch Schließen der Schließhilfe, indem die Scheiben aufeinander zu bewegt werden bis sie das Stoma zwischen Schließring und Basisplatte umfassen oder bis sie mit dem Schließring lösbar verbunden sind,
c. Lösen des Stomabeutels, indem eine Druckkraft auf die Scheiben der Schließhilfe und eine Zugkraft auf den Auffangbeutel angreift oder Anbringen eines Stomabeutels, indem eine Zugkraft auf die Scheiben und eine Druckkraft auf den Stomabeutel angreift.
d. Öffnen der Schließhilfe durch Auseinander bewegen der Scheiben,
e. Entfernen der Schließhilfe.

8. Verwendung einer Schließhilfe zum Anbringen oder Entfernen eines Auffangbeutels um ein Stoma eines Benutzers, wobei die Schließhilfe durch mindestens zwei gegeneinander durch Gelenk verschwenkbare oder durch eine Führung zueinander verschiebbare Scheiben besteht, die im zueinander bewegten Zustand das Stoma eines Benutzers umgreifend entweder zwischen Schließring und Stomabasisplatte anordenbar oder mit dem Schließring lösbar verbindbar sind, wobei die Scheiben über die äußeren Ränder der Basisplatte hinaus ragen und so stabil ausgebildet ist, dass angreifbare Kräfte beim Wechsel des Auffangbeutels auf die Gesamtfläche der Schreiben verteilt werden.

9. Verwendung einer Schließhilfe zum Anbringen oder Entfernen eines Auffangbeutels Vorrichtung zur Unterstützung des Verbindens eines Auffangbeutels mit einem Stomaverschluß, wobei die Schließhilfe nur temporär während des Anbringens oder Entfernens verwendet wird, wobei der Stomaverschluss einen Schließring aufweist, der auf einer Basisplatte befestigt ist, wobei die Basisplatte auf der Bauchdecke befestigt ist, **gekennzeichnet durch** mindestens eine flächige Scheibe, die U-förmig ausgebildet ist, so dass die Öffnung des U sich zwischen die Basisplatte und den Schließring legt oder mit dem Schließring eine stabile lösbare Verbindung eingeht, so dass ein temporäres Aufschieben ermöglicht wird, wobei die Scheiben über die äußeren Ränder der Basisplatte hinaus ragen und so stabil ausgebildet ist, dass angreifbare Kräfte beim Wechsel des Auffangbeutels auf die Gesamtfläche der Schreiben verteilt werden.

10. Verwendung einer Schließhilfe nach einem oder mehreren der vorhergehenden Verwendungsansprüche, wobei die lösbare Verbindung durch eine Nut- und Zapfenverbindung erfolgt, die Nut sich am Schließring und der Zapfen an den Scheiben oder die Nut sich an den Scheiben und der Zapfen am Schließring befindet."

11. Verwendung einer Schließhilfe nach einem oder mehreren der vorhergehenden Verwendungsansprüche **dadurch gekennzeichnet, dass** sich die Scheiben teilweise überlappen.

12. Verwendung einer Schließhilfe nach einem oder mehreren der vorhergehenden Verwendungsansprüche **dadurch gekennzeichnet, dass dadurch gekennzeichnet, dass** die Scheiben gegeneinander verschwenkbar sind und jeweils eine halbkreisförmige oder sichelförmige Aussparung aufweisen, wobei sich die halbkreisförmigen oder sichelförmigen Aussparungen im Gebrauchszustand zu einer, das Stoma eines Benutzers umfassenden, kreisförmigen Durchgangsöffnung ergänzen.

13. Verwendung einer Schließhilfe nach einem oder mehreren der vorhergehenden Verwendungsansprüche **dadurch gekennzeichnet, dass** die Scheiben abwaschbar sind und aus Kunststoff oder Metall bestehen.

14. Verwendung einer Schließhilfe nach einem oder mehreren der vorhergehenden Verwendungsansprüche **dadurch gekennzeichnet, dass** die Scheiben aus Pappe oder Papier besteht.

15. Verwendung einer Schließhilfe nach dem vorhergehenden Verwendungsanspruch, **dadurch gekennzeichnet, dass** das Papier ein Teil der Verpackung des Auffangbeutels ist.
